(19) Europäisches Patentamt
European Patent Office
Office européen des brevets

(11) Publication number: **0 050 558 B1**

# (12) EUROPEAN PATENT SPECIFICATION

(45) Date of publication of patent specification: **17.07.85**

(51) Int. Cl.$^4$: **G 03 C 1/485**

(21) Application number: **81401589.7**

(22) Date of filing: **14.10.81**

## (54) Photographic emulsions and elements capable of forming direct-positive images.

(30) Priority: **16.10.80 FR 8022103**
**17.11.80 US 207530**

(43) Date of publication of application:
**28.04.82 Bulletin 82/17**

(45) Publication of the grant of the patent:
**17.07.85 Bulletin 85/29**

(84) Designated Contracting States:
**BE DE FR GB**

(56) References cited:
**DE-A-1 908 570**
**DE-A-2 154 076**
**FR-A-2 320 290**
**FR-A-2 356 972**
**US-A-3 615 615**
**US-A-3 718 470**
**US-H- 967 002**

(73) Proprietor: **EASTMAN KODAK COMPANY**
**343 State Street**
**Rochester New York 14650 (US)**

(72) Inventor: **Compere, Marcel André**
**KODAK-PATHE 30, rue des Vignerons**
**F-94300 Vincennes (FR)**
Inventor: **Jordi, Frédéric Xavier**
**KODAK-PATHE 30, rue des Vignerons**
**F-94300 Vincennes (FR)**
Inventor: **Baralle, Roger Maurice**
**KODAK-PATHE 30, rue des Vignerons**
**F-94300 Vincennes (FR)**
Inventor: **Pfaff, Maurice Edgar**
**KODAK-PATHE 30, rue des Vignerons**
**F-94300 Vincennes (FR)**
Inventor: **Goumont, Claude Germain**
**KODAK-PATHE 30, rue des Vignerons**
**F-94300 Vincennes (FR)**

(74) Representative: **Parent, Yves et al**
**Kodak-Pathé Département des Brevets et**
**Licences 30, rue des Vignerons B.P. 60**
**F-94302 Vincennes Cedex (FR)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

EP 0 050 558 B1

**Description**

The present invention is directed to silver halide emulsions and photographic elements useful in forming direct-positive images. More specifically, this invention is directed to internal latent image-forming silver halide emulsions containing a nucleating agent.

A direct-positive image is understood in photography to be a positive image that is formed without first forming a negative image. Positive dye images which are not direct-positive images are commonly produced in color photography by reversal processing in which a negative silver image is formed and a complementary positive dye image is then formed in the same photographic element. The term "direct reversal" has been applied to direct-positive photographic elements and processing which produces a positive dye image without forming a negative silver image.

One conventional approach for obtaining direct-positive photographic images is to employ silver halide emulsions which are initially surface fogged. Surface fog is imagewise removed in exposed areas. When a photon is absorbed by a silver halide grain, a hole (a positive charge) and an electron are released in the silver halide crystal. Migration of the hole to the grain surface results in oxidation of a metallic silver atom (fog) to its ionic form, thereby reducing the developability of the grain. The present invention is totally inapplicable to surface-fogged direct-positive emulsions.

A second conventional approach to forming direct-positive images, to which the present invention relates, is to use photographic elements employing internal latent image-forming silver halide grains. After imagewise exposure, the silver halide grains are developed with a surface developer, that is, one which will leave the latent image sites within the silver halide grains substantially unrevealed. Simultaneously, either by uniform light exposure or by the use of a nucleating agent, the silver halide grains are subjected to development conditions that would cause fogging of a negative-working photographic element. The internal latent image-forming silver halide grains which received actinic radiation during imagewise exposure develop under these conditions at a comparatively slow rate, as compared to the internal latent image-forming silver halide grains not imagewise exposed. The result is a direct-positive silver image. In color photography, the oxidized developer that is produced during silver development is used to produce a corresponding positive, direct reversal dye image. Multicolor direct reversal photographic images have been extensively investigated in connection with image-transfer photography.

It has been found advantageous to employ nucleating agents in preference to uniform light exposure in the process described above. The term "nucleating agent" is employed herein to mean a fogging agent capable of permitting the selective development of internal latent image-forming silver halide grains which have not been imagewise exposed in preference to the development of silver halide grains having an internal latent image formed by imagewise exposure.

While nucleating agents have been long known to the photographic art, recent interest has focused on identifying nucleating agents that are effective in relatively low concentration levels and that can be incorporated directly into silver halide emulsions. Nucleating agents can be made more efficient if they can be made to closely associate with the silver halide grains. It is thus desirable to provide a new class of nucleating agents that adsorbs on the surface of silver halide grains.

This invention is directed to a silver halide emulsion comprised of silver halide grains capable of forming an internal latent image and a nucleating agent, characterized in that the nucleating agent is a compound of the formula (I)

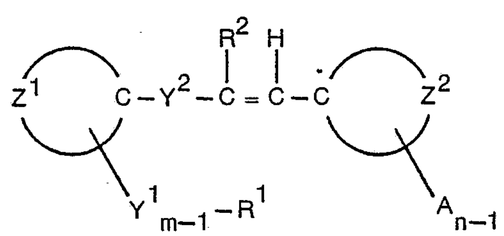

wherein

$Z^1$ represents the atoms completing an aromatic carbocyclic nucleus of from 6 to 10 nuclear carbon atoms;

$Y^1$ and $Y^2$ are independently selected from the group consisting of a divalent oxygen atom, a divalent sulfur atom and

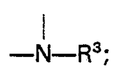

$Z^2$ represents the atoms completing a heterocyclic nucleus comprising a 5- or 6-membered ring containing at least one non-metallic heteroatom in the ring selected from the group consisting of oxygen, sulfur, selenium and nitrogen, the remaining ring atoms being carbon;

A is a group promoting the adsorption of the nucleating agent to the silver halide grains;

m and n are 1 or 2; and

$R^1$, $R^2$ and $R^3$ are independently selected from the group consisting of hydrogen, alkyl, aryl, alkaryl and aralkyl, and $R^1$ and $R^3$ are additionally independently selected from the group consisting of acyl, alkenyl, and alkynyl, the aliphatic portions of such groups containing up to 5 carbon atoms and the aromatic portions of such groups containing 6 to 10 carbon atoms.

The nucleating agents described above adsorb to the surface of silver halide grains like photographic spectral sensitizing dyes.

Photographic elements comprised of a support can be prepared with at least one layer of a silver halide emulsion as described above.

Referring to formula I, above, the nucleating agents employed in the practice of this invention are characterized by containing an aromatic carbocyclic nucleus of from 6 to 10 carbon atoms, such as a phenyl or naphthyl nucleus. In a preferred form $Z^1$ represents the atoms completing a phenyl nucleus. The aromatic carbocyclic nucleus is linked through a divalent chalcogen, such as divalent oxygen or sulfur atom, or a trivalent nitrogen to a vinylene group which is in turn bonded to a carbon atom of a heterocyclic nucleus of the type found in cyanine dyes. To facilitate description these nucleating agents are hereinafter referred to as vinylene nucleating agents.

The aromatic carbocyclic nucleus can be unsubstituted (e.g., phenyl or naphthyl), in which case $R^1$ is hydrogen and m is 1. The divalent vinylene group can also be unsubstituted, in which case $R^2$ is hydrogen. When $Y^2$ is

$$-\overset{|}{N}-R^3;$$

$R^3$, which satisfies the third valence of the nitrogen atom, can be hydrogen.

The aromatic carbocyclic nucleus, the trivalent nitrogen atom and the vinylene group can be substituted, each independently of the other. In preferred substituted forms, $R^1$, $R^2$, and $R^3$ can be selected from among alkyl, aryl, alkaryl and aralkyl substituents. For example, $R^1$, $R^2$, and $R^3$ can be independently selected from among alkyl groups, such as, methyl, ethyl, *n*-propyl, *i*-propyl, *n*-butyl, *i*-butyl, *t*-butyl, *n*-pentyl, *i*-pentyl, and *neo*-pentyl; aryl groups, such as, phenyl and naphthyl; alkaryl groups, such as, *p*-tolyl, *o*-tolyl, xylyl, and 2,3,5-trimethylphenyl; and aralkyl groups, such as benzyl and phenethyl. In further preferred forms $R^1$ and $R^3$ can additionally be selected independently from among acyl, alkenyl, and alkynyl substituents. Preferred acyl substituents are carboxyl substituents, such as, formyl, acetyl, propanoyl, butanoyl, and pentanoyl. Exemplary preferred alkenyl substituents are vinyl and allyl. Exemplary preferred alkynyl substituents are propargyl and 3-butynyl. In the foregoing preferred forms of $R^1$, $R^2$, and $R^3$, the aliphatic moieties contain up to 5 carbon atoms and the aryl moieties contain 6 to 10 carbon atoms. In a specifically preferred form, $R^1$, when present as a substituent, is linked to the aromatic carbocyclic nucleus through $Y^1$. $Y^1$ can be a divalent chalcogen, such as a divalent oxygen or sulfur atom, or

$$-\overset{|}{N}-R^3.$$

Linked to the vinylene group in formula I is a heterocyclic nucleus of the type found in cyanine dyes. That is, $Z^2$ completes a 5- or 6-membered heterocyclic ring containing at least one, typically one or two, non-metallic hetero atoms chosen from the group consisting of chalcogen, such as, oxygen, sulfur, and selenium, and nitrogen, the remaining ring atoms being carbon. In a preferred form a benzo group is fused to the heterocyclic ring.

In a preferred form, the heterocyclic nucleus containing $Z^2$ in the compound of formula I can be

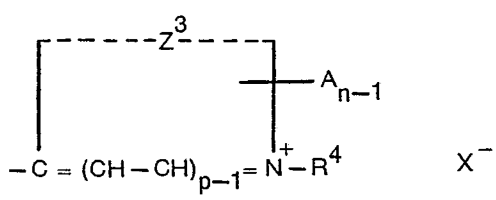

(II)

wherein; A is a group promoting the adsorption of the nucleating agent to the silver halide grains; n and p are 1 or 2; $R^4$ represents a ring nitrogen substituent of the type found in cyanine dyes; $Z^3$ complete a heterocyclic nucleus forming one 5- or 6-membered ring containing at least one non-metallic hetero atom in the ring chosen from the group consisting of oxygen, sulfur, selenium and nitrogen, the remaining ring atoms being carbon; and $X^-$ is an acid anion if required to satisfy charge neutrality.

An example of such a preferred form of the nucleating agent can correspond to the general formula

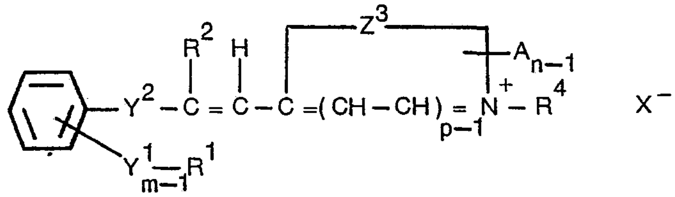

(IIa)

wherein $Y^1$, $Y^2$, A, $R^1$, $R^2$, $R^4$, $Z^3$, m, n and p and $X^-$ have been previously defined.

In preferred forms $Z^3$ represents the non-metallic atoms required to complete a heterocyclic nucleus having from 5- to 6-atoms in a heterocyclic ring, which can contain a second hetero atom, e.g., a hetero oxygen, sulfur, or selenium atom, or a second nitrogen atom, such as, a thiazole nucleus (e.g., thiazole, 4-methylthiazole, 4-phenylthiazole, 5-methylthiazole, 4,5-dimethylthiazole, and 4,5-diphenylthiazole), a benzothiazole nucleus (e.g., benzothiazole, 4-chlorobenzothiazole, 5-chlorobenzothiazole, 4-methylbenzothiazole, 6-methylbenzothiazole, 5-bromobenzothiazole, 5-methoxybenzothiazole, 6-iodobenzothiazole, and 5,6-dimethoxybenzothiazole), a naphthothiazole nucleus (e.g., α-naphthothiazole, β-naphthothiazole, 5-methoxy-β-naphthothiazole, 8-ethoxy-α-naphthothiazole, and β,β-naphthothiazole), a thianaphtheno-7′,6′,4,5-thiazole nucleus (e.g., 4′-methoxythianaphtheno-7′,6′,4,5-thiazole) an oxazole nucleus (e.g., 4-methyloxazole, 5-methyloxazole, 4-phenyloxazole, 4,5-diphenyloxazole, 4-ethyloxazole, and 4,5-diethyloxazole), a benzoxazole nucleus (e.g., benzoxazole, 5-chlorobenzoxazole, 5-methylbenzoxazole, 5-phenylbenzoxazole, 6-methylbenzoxazole, 5,6-dimethylbenzoxazole, 4,6-dimethylbenzoxazole, 5-ethoxybenzoxazole, 5,6-dichlorobenzoxazole, and 5-hydroxybenzoxazole), a naphthoxazole nucleus (e.g., α-naphthoxazole, β-naphthoxazole, and β,β-naphthoxazole), a selenazole nucleus (e.g., 4-methylselenazole and 4-phenylselenazole), a benzoselenazole nucleus (e.g., benzoselenazole, 5-chlorobenzoselenazole, 6-methoxybenzoselenazole, 5-hydroxybenzoselenazole, and a tetrahydrobenzoselenazole), a naphthoselenazole nucleus (e.g., α-naphthoselenazole, β-naphthoselenazole, and β,β-naphthoselenazole), a thiazoline nucleus (e.g., thiazoline and 4-methylthiazoline), a 2-pyridine nucleus (e.g., 2-pyridine and 5-methyl-2-pyridine), a 4-pyridine nucleus (e.g., 4-pyridine and 3-methyl-4-pyridine), a 2-quinoline nucleus (e.g., 2-quinoline, 3-methyl-2-quinoline, 5-ethyl-2-quinoline, 6-chloro-2-quinoline, 8-chloro-2-quinoline, 4-methoxy-2-quinoline and 8-hydroxy-2-quinoline, a 4-quinoline nucleus (e.g., 4-quinoline, 5-methyl-4-quinoline, 7-methyl-4-quinoline, and 8-chloro-4-quinoline), a 1-isoquinoline nucleus (e.g., 1-isoquinoline and 3,4-dihydro-1-isoquinoline), a 3-isoquinoline nucleus (e.g., 3-isoquinoline), a 3,3-dialkylindolenine nucleus (e.g., 3,3-dimethylindolenine, 3,3,5-trimethylindolenine, and 3,3,7-trimethylindolenine), an imidazole nucleus (e.g., imidazole, 1-alkylimidazole, 1-alkyl-4-phenylimidazole, and 1-alkyl-4,5-dimethylimidazole), a benzimidazole nucleus (e.g., benzimidazole, 1-alkylbenzimidazole, 1-arylbenzimidazole, and 5,6-dichlorobenzimidazole), or a naphthimidazole nucleus (e.g., 1-alkyl-α-naphthimidazole, 1-aryl-β-naphthimidazole, and 1-alkyl-5-methoxy-β-naphthimidazole).

$R^4$ can be any ring nitrogen substituent found in cyanine dyes. Such substituent is commonly an alkyl group having 1 to 18 carbon atoms, such as, methyl, ethyl, propyl, *i*-propyl, butyl, *i*-butyl, pentyl, hexyl, heptyl, octyl, nonyl, decyl, undecyl, dodecyl, tridecyl, tetradecyl, pentadecyl, hexadecyl, heptadecyl, and octadecyl. $R^4$ can alternatively be an aryl group (e.g., phenyl or naphthyl), an alkaryl group (e.g., tolyl, ethylphenyl, and xylyl), an arylkyl group (e.g., benzyl and phenethyl), or an alkenyl group (e.g., allyl, propenyl, 1-butenyl, and 2-butenyl). The above hydrocarbon groups when present in cyanine dyes are frequently in turn substituted with halogen atoms or groups such as hydroxy, alkoxy, carboxy, sulfo, sulfato, alkanoyloxy, and alkoxycarbonyl groups. It is generally preferred that the aliphatic moieties of the $R^4$ substituent contain 5 or fewer carbon atoms while the aromatic moieties contain 6 to 10 carbon atoms. The aromatic moieties are most preferably phenyl groups.

The nucleating agents used in this invention, which contain a cyanine dye heterocyclic nucleus, require no other adsorption promoting moiety to achieve effective association with silver halide grain surfaces. However, since higher degrees of adsorption to silver halide grain surfaces can reduce the concentration of nucleating agent required to produce efficient nucleation, it is recognized that an additional adsorption promoting group can be usefully incorporated into the nucleating agent.

The adsorption promoting group can be chosen from among those known to promote adsorption of photographic addenda to silver halide grain surfaces. For example, a wide variety of such groups are known and used in the photographic art to adsorb stabilizers, antifoggants, and sensitizing dyes to silver halide grain surfaces. Typically, such moieties contain a sulfur or nitrogen atom capable of complexing with silver or otherwise exhibiting an affinity for the silver halide grain surfaces. The adsorption promoting sulfur atom can be present in the form of a thiacarbonyl or mercapto group. Particularly useful mercapto groups are attached to a carbon atom of a carbocyclic or heterocyclic aromatic ring and may tautomerize to a thiacarbonyl form. Particularly useful thiacarbonyl groups can be present in acyclic groups, such as thiourea moieties, or in heterocyclic nuclei (typically 5- or 6-membered heterocyclic rings) of the type found in stabilizers, antifoggants and merocyanine spectral sensitizing dyes. Thio groups, such as found in thiazole nuclei exhibit sufficient affinity for silver halide grain surfaces to enhance adsorption. Nitrogen atom containing adsorption promoting moieties are typified by those containing a trivalent nitrogen atom with a displaceable hydrogen atom appended. The trivalent atom is usually a member of a 5- or 6-

membered heterocyclic ring of the type found in stabilizers, antifoggants and cyanine spectral sensitizing dyes.

The adsorption promoting group A can be any group which enhances the nucleating agent's capability of being absorbed to the silver halide grain surface. In structural terms, such groups can include adsorption promoting groups selected from the following classes:

nitrogen-containing heterocyclic groups bearing an ionizable hydrogen atom, for example, triazole and benzotriazole radicals, and in particular 1,2,3-benzotriazole-5-yl; tetrazole radicals, and in particular 5-tetrazolyl; benzimidazole radicals, particularly 1,3-benzimidazol-5-yl; imidazole, indazole, triazole, imidazo-tetrazole, and pyrazolotriazole radicals as well as hydroxytetraazindene and hydroxypentaazaindene radicals, such as 4-hydroxy-6-methyl-1,3,3a,7-tetraazaindene radicals;

heterocyclic groups having at least one ring nitrogen and at least one further ring atom which is oxygen, sulfur, or selenium, for example, thiazole, benzothiazole, naphthothiazole, thiazoline, thiazoline thione, thiazolidine, thiadiazole and rhodanine radicals as well as oxygen and selenium analogues thereof; for example, benzoxazole and benzoselenazole radicals;

groups bearing mercapto groups or groups which can tautomerize to the thiol form; for example, radicals of alkyl thiols or derivatives such as cysteine; or heterocyclic rings bearing a mercapto group, such as, benzothiazole-2-thiols, benzoxazole-2-thiols, 1-phenyl-5-mercaptotetrazoles, and 1,2,3-benzotriazine-4-thiones; and

groups containing the function

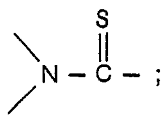

for example, radicals of alkyl or aryl substituted thioamides (including thioureas), thiohydantoins, thiobarbituric acids, and their derivatives; for example, thioacetamides or thioanilides, thiosemicarbazides, and dithiocarbamates.

In addition to a group of the type identified above capable of complexing with silver or otherwise producing an affinity for the silver halide grain surfaces, the adsorption promoting moiety can (but need not) include a divalent linking group of any form providing an attachment to the heterocyclic nucleus. Among specifically contemplated linkages are amido, sulfonamido, carbamoyl, sulfamoyl, and ester divalent linking groups, preferably in combination with divalent hydrocarbon or hydrocarbonoxy linking groups, such as, alkylene, alkyleneoxy, and phenylene groups, wherein the alkylene groups contain from 1 to 5 carbon atoms. Such divalent linking groups, when present, exhibit only a secondary influence on the activity of the compounds prepared. It is therefore apparent that such divalent linking groups can be chosen from among a wide variety of known linking groups.

A preferred class of adsorption promoting groups are thioamides of the formula

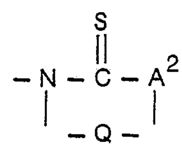

(III)

wherein

$A^2$ is $=N—R^5$, —S—, or —O—;

Q represents the atoms necessary to complete a 5-membered heterocyclic nucleus; and

$R^5$ is chosen from among hydrogen, phenyl, alkyl, alkylphenyl, and phenylalkyl, the alkyl groups in each instance containing from 1 to 5 carbon atoms.

The adsorption promoting groups have a 5-membered heterocyclic thioamide nucleus such as a 5-thiazoline-2-thione, thiazolidine-2-thione, 4-oxazoline-2-thione, oxazolidine-2-thione, 2-pyrazoline-5-thione, pyrazolidine-5-thione, indoline-2-thione, or 4-imidazoline-2-thione. A specifically preferred subclass of heterocyclic thioamide nuclei is formed when Q is as indicated in formula (IV)

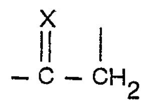

(IV)

wherein X is S or O.

Specifically preferred illustrations of such values of Q are 2-thiohydantoin, rhodanine, isorhodanine, and 2-thio-2,4-oxazolidinedione nuclei. It is believed that some 6-membered nuclei, such as thiobarbituric acid, may be equivalent to 5-membered nuclei embraced within formula (IV). These and more complex absorption promoting groups are disclosed in U.S. Patent 4,080,207.

The thioamide adsorption promoting groups useful in the practice of this invention need not form a

heterocyclic ring in order to be effective. For example, the thioamide group can take the form of a thiourea group. Preferred thiourea groups are those of the formula

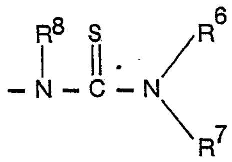
(V)

wherein

$R^6$, $R^7$, and $R^8$ can be independently selected from the group described above in connection with $R^2$, provided at least one of $R^6$, $R^7$ and $R^8$ is hydrogen and at least one of $R^6$ and $R^7$ is other than hydrogen, and $R^6$ and $R^7$ can together form a heterocyclic nucleus having a 5- or 6-membered ring, wherein the ring atoms are chosen from the class consisting of nitrogen, carbon, oxygen, sulfur, and selenium atoms. It is preferred that up to two of the ring atoms be hetero atoms and the remaining ring atoms be carbon atoms. The ring necessarily contains at least one nitrogen atom. Exemplary rings include morpholino, piperidino, pyrrolidinyl, pyrrolinyl, thiomorpholino, thiazolidinyl, 4-thiazolinyl, selenazolidinyl, 4-selenazolinyl, imidazolidinyl, imidazolinyl, oxazolidinyl, and 4-oxazolinyl rings. Specifically preferred rings are saturated or otherwise constructed to avoid electron withdrawal from the remaining nitrogen atom of the thiourea group.

The adsorption promoting group in another preferred form can be of the formula

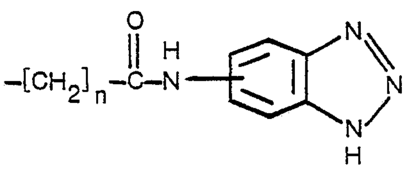
(VI)

wherein

n is an integer of 1 to 4.

The preferred adsorption promoting groups can be of the type described in U.S. Patents 4,030,925, 4,031,127 and 4,080,207 and Sidhu et al *Research Disclosure*, Vol. 176, December 1978, Item 17626.

Illustrative specific nucleating agents useful in the practice of this invention are those set forth below in Table I.

TABLE I

| | |
|---|---|
| NA—1 | 2-[β-anilinovinyl]-3-methylbenzothiazolium *p*-toluenesulfonate |
| NA—2 | 2-[β-(N-methylanilino)vinyl]-3-methylbenzo thiazolium *p*-toluenesulfonate |
| NA—3 | 2-[β-(*o*-methylthio-N-methylanilino)vinyl]-1-methylquinolinium *p*-toluenesulfonate |
| NA—4 | 2-[β-(N-propargylanilino)vinyl]-3-ethyl-benzoxazolium bromide |
| NA—5 | 2-[β-(*p*-N-methylaminophenoxy)vinyl]-3-methyl-benzothiazolium chloride |
| NA—6 | 2-[β-(*o*-methoxy-N-methylanilino)vinyl]-3-methylbenzothiazolium *p*-toluenesulfonate |
| NA—7 | 2-[β-(N-propargylanilino)vinyl]-3-ethylbenzo-selenazolium bromide |
| NA—8 | 1-ethyl-2-[β-N-propargylanilinovinyl]-1,3-diethyl-5,6-dichlorobenzimidazolium bromide |
| NA—9 | 2-[β-(*o*-propargylthio-N-propargylanilino)-vinyl]-3-methyl-benzothiazolium bromide |
| NA—10 | 2-[β-(*o*-methylthio-N-propargylanilino)vinyl]-3-methylbenzothiazolium *p*-toluenesulfonate |
| NA—11 | 2-[β-methyl-β-(*o*-propargylthio-N-methyl-anilino)vinyl]-3-methylbenzothiazolium bromide |
| NA—12 | 2-[β-N-propargylanilinovinyl]-3-methylbenzo-thiazolium bromide |
| NA—13 | 2-[β-(N-methylanilino)vinyl]-3-methyl-6-acetamidobenzothiazolium *p*-toluenesulfonate |
| NA—14 | 2-[-β-(N-methylanilino)vinyl]-3-methyl-6-(N'-ethylthioureido)benzothiazolium bromide |

The vinylene nucleating agents of the invention can be employed with any conventional photographic element capable of forming a direct-positive image containing, coated on a photographic support, at least one silver halide emulsion layer containing a vehicle and silver halide grains capable of forming an internal latent image upon exposure to actinic radiation. As employed herein, the terms "internal latent image silver halide grains" and "silver halide grains capable of forming an internal latent image" are employed in the art-recognized sense of designating silver halide grains which produce substantially higher optical densities when coated, imagewise exposed and developed in an internal developer than when comparably coated, exposed and developed in a surface developer. Preferred internal latent image silver halide grains are those which, when examined according to normal photographic testing techniques, by coating a test portion on a photographic support (e.g., at a coverage of from 3 to 4 grams per square meter), exposing to a light intensity scale (e.g., with a 500-watt tungsten lamp at a distance of 61 cm) for a fixed time (e.g., between $1 \times 10^{-2}$ and 1 second) and developing for 5 minutes at 25°C in Kodak (Registered Trademark) Developer DK-50 (a surface developer), provide a density of at least 0.5 less than when this testing procedure is repeated, substituting for the surface developer Kodak Developer DK-50 containing 0.5 gram per liter of potassium iodide (an internal developer). The internal latent image silver halide grains most preferred for use in the practice of this invention are those which, when tested using an internal developer and a surface developer as indicated above, produce an optical density with the internal developer at least 5 times that produced by the surface developer. It is additionally preferred that the internal latent image silver halide grains produce an optical density of less than 0.4 and, most preferably, less than 0.25 when coated, exposed and developed in surface developer as indicated above, that is, the silver halide grains are preferably initially substantially unfogged and free of latent image on their surface.

The surface developer referred to herein as Kodak Developer DK-50 is described in the *Handbook of Chemistry and Physics*, 30th edition, 1947, Chemical Rubber Publishing Company, Cleveland, Ohio, page 2558, and has the following composition:

| | |
|---|---|
| Water, about 125°F (52°C) | 500.0 cc |
| N-methyl-p-aminophenyl sulfate | 2.5 g |
| Sodium sulfite, desiccated | 30.0 g |
| Hydroquinone | 2.5 g |
| Sodium metaborate | 10.0 g |
| Potassium bromide | 0.5 g |
| Water to make | 1.0 liter |

Internal latent image silver halide grains which can be employed in the practice of this invention are well known in the art. Patents teaching the use of internal latent image silver halide grains in photographic emulsions and elements include U.S. Patents 2,592,250; 3,206,313; 3,761,266; 3,586,505; 3,772,030; 3,761,267 and 3,761,276.

The internal latent image silver halide grains preferably contain bromide as the predominant halide. The silver bromide grains can consist essentially of silver bromide or can contain silver bromoiodide, silver chlorobromide, silver chlorobromoiodide crystals and mixtures thereof. Internal latent image-forming sites can be incorporated into the grains by either physical or chemical internal sensitization. Chemical formation of internal latent image-forming sites can be produced through the use of sulfur, gold, selenium, tellurium and/or reduction sensitizers of the type described, for example, in U.S. Patents 1,623,499; 2,399,083; 3,297,447 and 3,297,446. Internal latent image sites can also be formed through the incorporation of metal dopants, particularly Group VIII noble metals, such as, ruthenium, rhodium, palladium, iridium, osmium and platinum, as taught by U.S. Patent 3,367,778. The preferred foreign metal ions are polyvalent metal ions which include the above-noted Group VIII dopants, as well as polyvalent metal ions such as lead, antimony, bismuth, and arsenic. In other preferred embodiments, the silver halide grains may be formed in the presence of bismuth, lead or iridium ions. In a preferred approach, the internal latent image sites can be formed within the silver halide grains during precipitation of silver halide. In an alternate approach, a core grain can be formed which is treated to form the internal image sites and then a shell deposited over the core grains.

The silver halide grains employed in the practice of this invention are preferably monodispersed, and in some embodiments are preferably large-grain emulsions made according to DE—A—2,107,118. The monodispersed emulsions are those which comprise silver halide grains having a substantially uniform diameter. Generally, in such emulsions, no more than 5 percent by number of the silver halide grains smaller than the mean grain size and/or no more than 5 percent by number of the silver halide grains larger than the mean grain size vary in diameter from the mean grain diameter by more than about 40 percent. Preferred photographic emulsions of this invention comprise silver halide grains, at least 95 percent by weight of said grains having a diameter which is within 40 percent and preferably within 30 percent of the mean grain diameter. Mean grain diameter, i.e., average grain size, can be determined using conventional methods, e.g., such as projective area, as shown in an article by Trivelli and Smith entitled "Empirical Relations Between Sensitometric and Size-Frequency Characteristics in Photographic Emulsion Series" in *The Photographic Journal*, Volume LXXIX, 1939, pages 330 through 338. The aforementioned uniform size distribution of silver halide grains is a characteristic of the grains in monodispersed photographic silver halide emulsions. Silver halide grains having a narrow size distribution can be obtained by controlling the conditions at which the silver halide grains are prepared using a double run procedure. In such a procedure, the silver halide grains are prepared by simultaneously running an aqueous solution of a silver salt, such as silver nitrate, and an aqueous solution of a water-soluble halide, for example, an alkali metal halide such as potassium bromide, into a rapidly agitated aqueous solution of a silver halide peptizer, preferably gelatin, a gelatin derivative or some other protein peptizer. Suitable methods for preparing photographic silver halide emulsions having the required uniform particle size are disclosed in an article entitled "Ia: Properties of Photographic Emulsion Grains", by Klein and Moisar, *The Journal of Photographic Science*, Volume 12, 1964, pages 242 through 251; an article entitled "The Spectral Sensitization of Silver Bromide Emulsions on Different Crystallographic Faces", by Markocki, *The Journal of Photographic Science*, Volume 13, 1965, pages 85 through 89; an article entitled "Studies on Silver Bromide Sols, Part I. The Formation and Aging of Monodispersed Silver Bromide Sols", by Ottewill and Woodbridge, *The Journal of Photographic Science*, Volume 13, 1965, pages 98 through 103; and an article entitled "Studies on Silver Bromide Sols, Part II. The Effect of Additives on the Sol Particles", by Ottewill and Woodbridge, *The Journal of Photographic Science*, Volume 13, 1965, pages 104 through 107.

Where internal latent image sites have been formed through internal chemical sensitization or the use of metal dopants, the surface of the silver halide grains can be sensitized to a level below that which will produce substantial density in a surface developer, that is, less than 0.4 (preferably less than 0.25) when coated, exposed and surface developed as described above. The silver halide grains are preferably predominantly silver bromide grains chemically surface sensitized to a level which would provide a maximum density of at least 0.5 using undoped silver halide grains of the same size and halide composition when coated, exposed and developed as described above.

The photographic silver halide emulsion layers and other layers of the photographic elements can contain various colloids alone or in combination as vehicles. Suitable hydrophilic materials include both naturally occurring substances such as proteins, protein derivatives, cellulose derivatives, e.g., cellulose esters, gelatin, e.g., alkali-treated gelatin (cattle bone or hide gelatin) or acid-treated gelatin (pigskin gelatin), gelatin derivatives, e.g., acetylated gelatin, phthalated gelatin, polysaccharides such as dextran, gum arabic, zein, casein, pectin, collagen derivatives, collodion, agar-agar, arrowroot, albumin, etc.

The photographic emulsions of the invention can be coated on a variety of supports. Typical photographic supports include cellulose acetate film, polymeric film such as poly(ethylene phthalate) and polystyrene film, wood fiber, e.g., paper, metallic sheet and foil, glass and ceramic supporting elements provided with one or more subbing layers to enhance the adhesive, antistatic, dimensional, abrasive, hardness, frictional, antihalation, and/or other properties of the support surface.

The vinylene nucleating agents of this invention can be employed in any desired concentration that will permit a degree of selectivity in developing imagewise silver halide grains capable of forming an internal latent image, which grains have not been imagewise exposed, as compared to silver halide grains containing an internal latent image formed by imagewise exposure.

It is preferred to incorporate the vinylene nucleating agents into the silver halide emulsions in concentrations of from $10^{-5}$ to $10^{-2}$ mole per mole of silver halide. Where an efficient adsorption promoting moiety is incorporated in the vinylene nucleating agent, such as a thioamide (particularly a thiourea) or a triazole, as described above, it is generally unnecessary to provide nucleating concentrations in excess of $10^{-3}$ mole per mole of silver halide. On the other hand, where such adsorption promoting nucleating agents are absent and the heterocyclic nucleus is being relied upon to promote adsorption to the silver halide grains, it is generally preferred that the nucleating agents be incorporated in a concentration of at least $10^{-4}$ mole per mole of silver halide. Where the vinylene nucleating agent is to be adsorbed to the surface of the silver halide grains, it can be adsorbed using the procedures well known to those skilled in the art for adsorbing sensitizing dyes, such as, cyanine and merocyanine dyes, to the surface of silver halide grains.

A simple exposure and development process can be used to form a direct-positive image. In one embodiment, a photographic element comprising at least one layer of a silver halide emulsion as described above can be imagewise exposed to light and then developed in a silver halide surface developer.

It is understood that the term "surface developer" encompasses those developers which will reveal the surface latent image on a silver halide grain, but will not reveal substantial internal latent image in an internal image-forming emulsion, and under the conditions generally used develop a surface-sensitive silver halide emulsion. The surface developers can generally utilize any of the silver halide developing agents or reducing agents, but the developing bath or composition is generally substantially free of a silver halide solvent (such as water-soluble thiocyanates, water-soluble thioethers, thiosulfates, and ammonia) which will disrupt or dissolve the grain to reveal substantial internal image. Low amounts of excess halide are sometimes desirable in the developer or incorporated in the emulsion as halide-releasing compounds, but high amounts of iodide or iodide-releasing compounds are generally avoided to prevent substantial disruption of the grain. Typical silver halide developing agents which can be used in the developing compositions of this invention include hydroquinones, catechols, aminophenols, 3-pyrazolidones, ascorbic acid and its derivatives, reductones and color developing agents, that is, primary aromatic amine developing agents, such as, aminophenols and *para*-phenylenediamines. The color developing agents are preferably employed in combination with black-and-white developing agents capable of acting as electron transfer agents.

Where the developing agents are initially entirely incorporated in the photographic elements, the remaining components (e.g., water, activators to adjust pH, preservatives, etc.) normally present in surface developers constitute what is commonly referred to as an activator solution. Except for the omission of the developing agent, activator solutions are identical to developer solutions in composition and are employed identically with incorporated developing agent photographic elements. Subsequent references to developing compositions are inclusive of both developer and activator solutions.

The surface developers are alkaline. Conventional activators, preferably in combination with buffers, such as, sodium hydroxide, potassium hydroxide, sodium carbonate, potassium carbonate, trisodium phosphate or sodium metaphosphate, can be employed to adjust pH to a desired alkaline level. The amounts of these materials present are selected so as to adjust the developer to a pH in the range of from 10 to 13, preferably from 10.2 to 12.0.

The developing compositions used to process the emulsions of this invention can contain certain antifoggants and development restrainers, or, optionally, they can be incorporated in layers of the photographic element.

In preferred photographic applications, the emulsions and elements can contain additional features which are in themselves well known to those familiar with the photographic arts. Further, these applications can entail conventional modifications in the procedures described above. A variety of such features are disclosed in *Research Disclosure*, Volume 176, December 1978, Item 17643, particulary Paragraph II, Emulsion washing; Paragraph IV, Spectral sensitization and Desensitization; Paragraph V, Brighteners; Paragraph VI, Antifoggants and stabilizers; Paragraph VIII, Absorbing and scattering materials; Paragraph X, Hardeners; Paragraph XI, Coating aids; Paragraph XII, Plasticizers and lubricants; Paragraph XIII, Antistatic layers; Paragraph XIV, Methods of addition; Paragraph XV, Coating and drying Procedures; Paragraph XVI, Matting agents; Paragraph XVIII, Exposure; Paragraph XIX, Processing (as applied to post-development processing and paragraphs G, H, I, and J, relating to amplification); Paragraph XX, Developing agents; and Paragraph XXI, Development modifiers.

It is specifically contemplated that the vinylene nucleating agents used in the present invention can be employed in combination with conventional nucleating agents of the quaternary ammonium salt, hydrazine, hydrazide, and hydrazone type. The conventional nucleating agents can be incorporated in the photographic element in previously taught concentrations, typically up to 2 grams per mole of silver. The conventional nucleating agents can also be present in the developer in previously taught concentrations, typically up to 5 grams per liter. Since the use of conventional nucleating agents is not essential to the practice of this invention, no minimum concentration is required; however, when employed, conventional nucleating agents are preferably present in a concentration range of from 10 to 500 mg per mole of silver when present in the emulsion and from 0.1 to 1 gram per liter when present in the developer.

The silver halide emulsions can be spectrally sensitized with cyanine, merocyanine, and other polymethine dyes and supersensitizing combinations thereof well known in the art. Spectral sensitizers in conventional surface-sensitive emulsions are comparably effective in the emulsions of this invention.

Photographic elements containing the emulsions of the invention are preferably color photographic elements which form dye images through the selective destruction, formation, or physical removal of dyes.

The emulsions of this invention are particularly useful with photographic elements used in image transfer processes or in image transfer film units. In a preferred image transfer system according to this invention employing negative-working dye image-providing compounds, a cross-oxidizing developing agent (electron transfer agent) develops silver halide and then cross-oxidizes with a compound containing a dye linked through an oxidizable sulfonamido group, such as a sulfonamidophenol, sulfonamidoaniline, sulfonamidoanilide, sulfonamidopyrazolobenzimidazole, sulfonamidoindole or sulfonamidopyrazole. Following cross-oxidation, hydrolytic deamidation cleaves the mobile dye with the sulfonamido group attached. Such systems are illustrated by U.S. Patents 3,928,312; 4,053,312 and U.S. Patent 4,076,529; U.K. Patent 1,489,694; DE—A—2,729,820; DE—A—2,613,005 and DE—A—2,505,248; and Kestner et al *Research Disclosure*, Volume 151, November 1976, Item 15157. Also specifically contemplated are otherwise similar systems which employ an immobile, dye-releasing (a) hydroquinone, as illustrated by U.S. Patent 3,698,897 and U.S. Patent 3,725,062, (b) *para*-phenylenediamine, as illustrated by Canadian Patent 602,607, or (c) quaternary ammonium compound, as illustrated by U.S. Patent 3,728,113.

Image transfer film units containing emulsions of this invention typically comprise:

(1) a photographic element comprising a support having thereon at least one silver halide emulsion layer containing radiation-sensitive internal latent image silver halide grains and a vinylene nucleating agent, the emulsion layer preferably having in contact therewith an image dye-providing material,

(2) an image-receiving layer, which can be located on a separate support and superposed or adapted to be superposed on the photographic element or, preferably, can be coated as a layer in the photographic element,

(3) an alkaline processing composition,

(4) means containing and adapted to release the alkaline processing composition into contact with the emulsion layer, and

(5) a silver halide developing agent located in at least one of the photographic element and alkaline processing composition so that the processing composition and developing agent, when brought together, form a silver halide surface developer.

The term "image dye-providing material", as used herein, is understood to refer to those compounds which are employed to form dye images in photographic elements. These compounds include dye developers, shifted dyes, color couplers, oxichromic compounds, dye redox releasers, etc, as described above in connection with positive-working and negative-working image transfer systems, such as disclosed in "Research Disclosure" item 17,643, p.30 (December 1975).

In a specific preferred form the photographic elements of this invention are intended to produce multicolor images which can be viewed in the elements or in a receiver when the elements form a part of a multicolor image transfer system. For multicolor image formation at least three superimposed color-forming layer units are coated on a support. Each of the layer units is comprised of at least one silver halide emulsion layer. At least one of the silver halide emulsions layers, preferably at least one of the silver halide emulsion layers in each color-forming layer unit and most preferably each of the silver halide emulsion layers, contains an emulsion according to this invention substantially as described above. The emulsion layers of one of the layer units are primarily responsive to the blue region of the spectrum, the emulsion layers of a second of the layer units are primarily responsive to the green region of the spectrum, and the

emulsion layers of a third of the layer units are primarily responsive to the red region of the spectrum. The layer units can be coated in any conventional order. In a preferred layer arrangement the red responsive layer unit is coated nearest the support and is overcoated by the green responsive layer unit, a yellow filter layer and a blue responsive layer unit. The layer units each contain in the emulsion layers or in adjacent hydrophilic colloid layers at least one image dye-providing compound. Such compounds can be selected from among those described above. Incorporated dye-forming couplers and redox dye-releasers constitute exemplary preferred image dye providing compounds. The blue, green and red responsive layer units preferably contain yellow, magenta and cyan image dye providing compounds, respectively.

The preparation of compounds useful as nucleating agents in the practice of this invention is generally known. A description of the methods used for the synthesis of such compounds is given for example in F. M. Hamer's book, *The Cyanine Dyes and Related Compounds*, Interscience Publishers, John Wiler & Sons, New York, 1964 (pp 447 *et seq.*). These methods can be used to prepare the nucleating agents used in the invention.

A few examples of preparation of compounds useful as nucleating agents are given hereafter.

*Compound NA-1*

2-[β-anilinovinyl]-3-methylbenzothiazolium p-toluenesulfonate

112 g of 2,3-dimethyl-benzothiazolium *p*-toluenesulfonate were introduced into 200 ml of xylene. The mixture was heated to 70—80°C and a previously prepared mixture of 75 g of ethyl *o*-formate, 93 g of aniline and 50 ml of xylene and 1 ml of sulfuric acid as a reaction catalyst was added with good stirring. An orange colored product was obtained (Yield: 80%, m.p. 244°C).

Analysis: $C_{23}H_{22}N_2O_3S_2$

| Percent: | C | H | N | S |
|---|---|---|---|---|
| Calculated: | 63.01 | 5.02 | 6.39 | 14.61 |
| Found: | 62.18 | 4.83 | 6.23 | 16.01 |

*Compound NA-2*

2-[β-(N-methylanilino)vinyl]-3-methylbenzothiazolium *p*-toluenesulfonate

To 100 ml of acetone was added 13.3 g of 3-methyl-2-β-anilinoethylidene benzothiazoline. The mixture was stirred, then 10 g of methyl *p*-toluenesulfonate were added. The reagents were left in contact for 72 hours. A solid was formed, collected and drained, then recrystallized from a mixture of ethanol and ether (m.p. 138°C).

Analysis: $C_{24}H_{24}N_2O_3S_2$ (mol. weight: 452)

| Percent: | C | H | N |
|---|---|---|---|
| Calculated: | 63.71 | 5.38 | 6.19 |
| Found: | 60.66 | 5.45 | 5.92 |

*Compound NA-3*

2-[β-(*o*-methylthio-N-methylanilino)vinyl]-1-methylquinolinium *p*-toluenesulfonate

15.2 g of 1-methyl-2-(3-methyl-2-benzothiazolinylidene)quinolinium *p*-toluenesulfonate were dissolved in 120 ml of toluene. 10 g of methyl *p*-toluenesulfonate were added. The precipitate formed was drained and recrystallized from methanol.

Analysis: $C_{27}H_{28}N_2O_3S_2$ (mol. weight: 492)

| Percent: | C | H | N | S |
|---|---|---|---|---|
| Calculated: | 65.85 | 5.69 | 5.69 | 13.00 |
| Found: | 62.73 | 5.70 | 5.37 | 13.07 |

*Compound NA-4*

2-[β-(N-propargylanilino)vinyl]-3-ethylbenzoxazolium bromide

13.2 g of β-anilinoethylidene ethyl-3-benzoxazoline were dissolved in 250 ml of acetone; 6 ml of propargyl bromide were added at room temperature. Crystals were formed after 2 hours of contact. Stirring was continued for 24 hours. The product was filtered off and recrystallized from a methanol/ether mixture (m.p. 198°C).

Analysis: $C_{20}H_{19}BrN_2O$ (mol. weight: 383)

| Percent: | C | H | N |
|---|---|---|---|
| Calculated: | 62.66 | 4.96 | 7.30 |
| Found: | 61.76 | 4.75 | 7.20 |

*Compound NA-5*

2-[β-(*p*-N-methylaminophenoxy)vinyl-3-methylbenzothiazolium chloride

To 200 ml of ethanol were added 34.4 g of *p*-hydroxymethylaminobenzene sulfate and 11.2 g of potassium hydroxide. This mixture was stirred for 30 minutes, its temperature being maintained between 5—10°C. 40.6 g of 2-(N-benzoylanilinovinyl)-3-methylbenzothiazolium chloride were added. A solubilization occurred first, then a precipitation while a strong red color was formed. The product was drained, washed with acetone and recrystallized from a methanol/ether mixture (m.p. of the purified product 262°C).

Analysis: $C_{17}H_{17}ClN_2OS$ (mol. weight: 332.5)

| Percent: | C | H | N | Cl | S |
|---|---|---|---|---|---|
| Calculated: | 61.35 | 5.11 | 8.42 | 10.67 | 9.67 |
| Found: | 59.15 | 5.12 | 8.36 | 10.77 | 9.52 |

*Compound NA-6*

2-[β-(*o*-methoxy-N-methylanilino)vinyl]-3-methylbenzothiazolium *p*-toluenesulfonate

To 250 ml of ethanol were added 46.8 g of 2-[β-(*o*-methoxyanilino)vinyl]-3-methylbenzothiazolium *p*-toluenesulfonate; 10 g of pelleted potassium hydroxide were added and the mixture was stirred for 30 minutes at room temperature. A gummy material was obtained.

500 ml of water was added; the half-gummy half-crystalline material formed was filtered and dissolved in 500 ml of toluene; it was dried on potassium hydroxide, filtered and 18.6 g of methyl *p*-toluene-sulfonate (0.09 mole) were added to the toluene solution. The mixture was allowed to rest for 24 hours; a precipitate was formed and filtered, then recrystallized from a methanol/ether mixture (m.p. of the purified product: 188°C).

Analysis: $C_{25}H_{26}N_2O_4S_2$ (mol. weight: 482)

| Percent: | C | H | N | S |
|---|---|---|---|---|
| Calculated: | 62.24 | 5.39 | 5.80 | 13.2 |
| Found: | 61.85 | 5.20 | 5.79 | 14.4 |

*Compound NA-7*

2-[β-(N-propargylanilino)vinyl]-3-ethylbenzoselenazolium bromide

In 200 ml of ethanol were dissolved 5.03 g of 3-ethyl-2-anilinovinylbenzoselenazolium *p*-toluene-sulfonate. 1 g of pelleted potassium hydroxide was added at room temperature and stirring was continued for 4 hours. The precipitate was drained and extracted with benzene. To the benzene solution were added 2 g of propargyl bromide which were left in contact for 72 hours. The precipitate formed was drained and recrystallized from a methanol/ether mixture (m.p. of the product thus purified: 200°C).

Analysis: $C_{20}H_{19}BrN_2Se$ (mol. weight: 446)

| Percent: | C | H | N |
|---|---|---|---|
| Calculated: | 53.81 | 4.26 | 6.27 |
| Found: | 53.59 | 4.32 | 6.28 |

*Compound NA-8*

2-[β-(N-propargylanilino)vinyl]-1,3-diethyl-5,6-dichloro benzimidazolium bromide

11.4 g of 1,3-diethyl-5,6-dichloro-2-anilinovinyl-benzimidazolium *p*-toluenesulfonate were added to 25 ml of ethanol. 2 g of pelleted potassium hydroxide were added and the mixture (sparingly soluble at the beginning of the reaction) was stirred and slowly solubilized. Then the base separated. It was drained after two hours of contact and extracted with toluene. 10 g of propargyl bromide were added to the toluene solution, then it was stirred for 72 hours at room temperature; the product was drained and recrystallized from a methanol/ether mixture (m.p. of the purified product: 144°C).

Analysis: $C_{22}H_{22}BrCl_2N_3—CH_3OH$ (mol. weight: 511)

| Percent: | C | H | N |
|---|---|---|---|
| Calculated: | 54.11 | 5.09 | 8.21 |
| Found: | 53.02 | 5.14 | 8.29 |

*Compound NA-9*

2-[β-(*o*-propargylthio-N-propargylanilino)vinyl]-3-methylbenzothiazolium bromide

In 20 ml of acetone, were dissolved 2 g of 3-propargyl-2-[(methyl-3'-benzothiazolinylidene)methyl]benzothiazoline. 2 ml of propargyl bromide were added. A precipitate was rapidly formed. The mixture was stirred and cooled, then the precipitate was filtered out and washed with acetone. The material was recrystallized from a methanol/ether mixture.

Analysis: $C_{22}H_{19}BrN_2S_2$ (mol. weight: 455)

| Percent: | C | H | N |
|---|---|---|---|
| Calculated: | 58.02 | 4.19 | 6.15 |
| Found: | 57.52 | 4.18 | 6.12 |

*Compound NA-10*

2-[β-(*o*-methylthio-N-propargylanilino)vinyl]-3-methyl-benzothiazolium *p*-toluenesulfonate

The procedure described for the synthesis of Compound NA-9 was used, but methyl *p*-toluenesulfonate was substituted in place of propargyl bromide.

Analysis: $C_{27}H_{26}N_2O_3S_3$ (mol. weight: 522)

| Percent: | C | H | N |
|---|---|---|---|
| Calculated: | 62.06 | 4.98 | 5.36 |
| Found: | 61.90 | 4.94 | 5.15 |

*Compound NA-11*

2-[β-methyl-β-(*o*-propargylthio-N-methylanilino)vinyl]-3-methylbenzothiazolium bromide

To 20 ml of benzene, were added 3.12 g of dimethyl-2,3-[(3'-methyl-benzothiazolinylidene)methyl]-2-benzothiazoline (1/100 mol.). The suspension was stirred at room temperature. 4 ml of propargyl bromide were added. A gum was formed. After 2 hours rest, benzene was decanted and the residue was washed with acetone.

The residue was dissolved in ethanol and filtered. The filtrate was poured into hexane and the product crystallized out; it was drained and washed with hexane (m.p. 170°C).

Analysis: $C_{21}H_{21}BrN_2S_2$ (mol. weight: 455)

| Percent: | C | H | N |
|---|---|---|---|
| Calculated: | 56.63 | 4.71 | 6.29 |
| Found: | 54.19 | 4.63 | 6.01 |

*Compound NA-12*

2-[β-(N-propargylanilino)vinyl]-3-methylbenzothiazolium bromide

26.6 g of N-anilinoethylidene methyl-3-benzothiazoline were suspended in 250 ml of acetone. 13 g of propargyl bromide were added in one portion. The mixture was stirred for 72 hours at room temperature. The precipitate formed was filtered out and recrystallized from a methanol/acetone/ether mixture. The product was drained and washed with acetone.

Analysis: $C_{19}H_{17}BrN_2S$ (mol. weight: 385)

| Percent: | C | H | N |
|---|---|---|---|
| Calculated: | 59.22 | 4.41 | 7.27 |
| Found: | 58.61 | 4.31 | 7.25 |

*Compound NA-13*

2-[β-(N-methylanilino)vinyl]-3-methyl-6-acetamidobenzothiazolium *p*-toluenesulfonate

A solution of 6.05 g of ethyl *o*-formiate and 3.8 g of aniline in 50 ml of xylene containing a trace of concentrated sulfuric acid was added to a suspension of 14 g of 2,3-dimethyl-6-acetamidobenzothiazolium *p*-toluenesulfonate. The mixture was heated for 4 hours at 80°C; it was completely dissolved, then reprecipitated. The solid formed (13 g, yield: 73.5%) was filtered out, washed with acetone and recrystallized from methanol. To a solution of 6.5 g of the preceding recrystallized material in 65 ml of methanol were added 4 g of 30% sodium methylate. The solution left for 5 hours at room temperature became green, the precipitate formed was filtered out, washed with water and acetone, extracted with 200 ml of benzene, and to the concentration of the benzene was added 65 ml of acetone and 3 g of methyl *p*-toluenesulfonate. The solution was refluxed for 2 hours. The yellow product (4 g, yield: 60%) which precipitated was filtered out and washed with ether.

*Compound NA-14*

2-[β-(N-methylanilino)-vinyl]-3-methyl-6-(N′-ethylthioureido)benzothiazolium bromide

A suspension of 2.475 g of NA-13 in 6 ml of 25% hydrochloric acid was refluxed for 1 hour. After solubilization, then reprecipitation, 0.6 g of potassium bromide were added and stirring was continued for 1 hour at room temperature. After neutralization with an aqueous solution of sodium hydroxide, the precipitate (1.6 g, yield: 87.5%) was filtered out and dried. 0.55 g of ethyl isothiocyanate were added to a solution of 1.47 g of the preceding filtered material in 150 ml of ethanol. The solution was refluxed for 1 hour 30 minutes under a nitrogen atmosphere. The precipitate formed was filtered out and washed with ether (1.1 g, yield 60%).

The following examples illustrate the use of the vinylene compounds as nucleating agents for internal latent image-forming direct-positive emulsions.

Examples 1 through 3

A gelatino-silver bromide emulsion capable of forming an internal latent image was employed. The emulsion was internally sulfur and gold sensitized and surface sulfur and gold sensitized to a lesser extent to provide a high ratio of internal to surface chemical sensitization. The emulsion contained cubic silver bromide grains having an average diameter of 0.6 micrometer. (The average grain diameter is the diameter of a circle equal in area to the mean projected area of the silver halide grains).

To various samples of the emulsion were added a nucleating agent as identified in Table II. Each emulsion sample was separately coated on poly(ethylene terephthalate) photographic film support at a coverage of 125 mg/dm$^2$ of gelatin and 50 mg/dm$^2$ of silver.

The resulting photographic elements were then given a sensitometric exposure with a light source having a temperature of 2850°K. A sample of each element was developed in Developer A below and another sample was developed in Developer B below. Both developers were surface developers; that is, they did not develop the internal latent image. Both developers produced direct-reversal images.

<u>Developer A</u>

| | |
|---|---|
| Elon (p-methylaminophenol, sulfate) | 1 g |
| Ascorbic acid | 10 g |
| Sodium metaborate | 40 g |
| Water to make | 1 liter |
| pH 10.2 | |

<u>Developer B</u>

| | |
|---|---|
| Elon (p-methylaminophenol, sulfate) | 1 g |
| Ascorbic acid | 10 g |
| Sodium metaborate | 40 g |
| Benzotriazole | 0.1 g |
| Water to make | 1 liter |
| pH adjusted to 12.3 with KOH | |

TABLE II

| Nucleating Agent | | Developer A | | | Developer B | | |
|---|---|---|---|---|---|---|---|
| Compound No. | Amount (mg/Ag mole) | Development Time (minutes) | $D_{min}$ | $D_{max}$ | Development Time (minutes) | $D_{min}$ | $D_{max}$ |
| NA–9 | 200 | 16 | 0.08 | 2.60 | 8 | 0.23 | 2.00 |
| NA–12 | 150 | 14 | 0.08 | 2.45 | 8 | 0.15 | 2.30 |
| NA–11 | 200 | 20 | 0.06 | 1.95 | 9 | 0.12 | 2.30 |

Examples 4 and 5

An internal image silver bromide emulsion was prepared by the procedure of Example 1. To a first sample of this emulsion were added 400 mg per silver mole of Compound NA-2, and to a second sample were added 40 mg per silver mole of Compound NA-14. These two emulsion samples were coated on a polyethylene-coated photographic paper support at a coverage of 5 mg/dm² of silver and 27.5 mg/dm² of gelatin. This emulsion contained 6 mg/dm² of a yellow dye-forming coupler having the following formula:

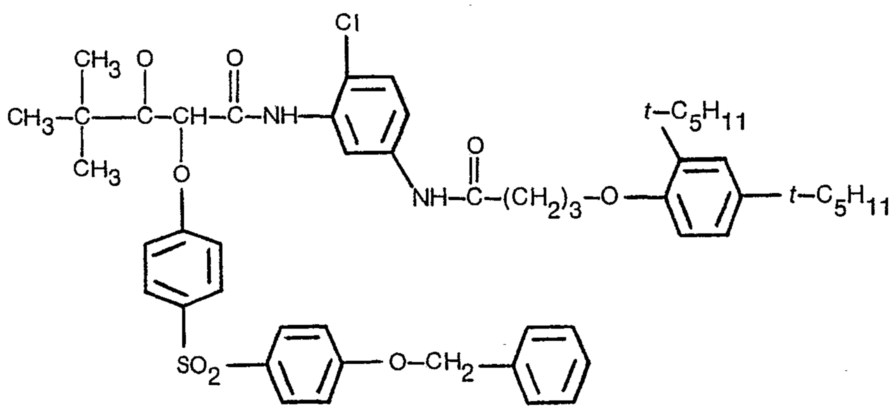

A gelatin layer was then coated over each emulsion layer.

Each sample was exposed as in Example 1, then processed for 4 minutes at 38°C in the surface developer having the following composition:

Developer C

| | |
|---|---|
| Benzyl alcohol | 16 ml |
| N-ethyl-N-methyl-sulfonamidoethyl-2-methyl-*p*-phenylenediamine sesquisulfate | 6 g |
| Piperidino hexose reductone | 0.5 g |
| Sodium sulfite | 1.5 g |
| Potassium carbonate | 30 g |
| Benzotriazole | 10 mg |
| Water to make | 1 liter |

pH: 10.5

Processing was finished as follows:

| | |
|---|---|
| Stop bath | 20 seconds |
| Bleach-fixing | 1 minute |
| Washing | 2 minutes. |

In both cases a yellow positive image was obtained whose characteristics are given in Table III.

TABLE III

| Nucleating Agent | Amount mg/mole Ag | $D_{min}$ | $D_{max}$ |
|---|---|---|---|
| NA–2 | 400 | 0.14 | 1.90 |
| NA–14 | 40 | 0.12 | 2.05 |

When the procedure described above was repeated with a third sample lacking vinylene compound incorporated as a nucleating agent, substantially no positive image was discernible at the end of 4 minutes, but after 12 minutes a positive image was visible. Thus, the nucleating agents of the present invention are capable of significantly accelerating the formation of a positive image.

**Claims**

1. A silver halide emulsion comprised of silver halide grains capable of forming an internal latent image and a nucleating agent, characterized in that said nucleating agent is a compound of the formula

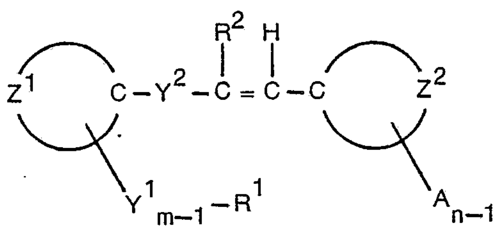

wherein

$Z^1$ represents the atoms completing an aromatic carbocyclic nucleus of from 6 to 10 nuclear carbon atoms;

$Y^1$ and $Y^2$ are independently selected from the group consisting of a divalent oxygen atom, a divalent sulfur atom and

$$-N-R^3;$$

$Z^2$ represents the atoms completing a heterocyclic nucleus comprising a 5- or 6-membered ring containing at least one non-metallic heteroatom in the ring selected from the group consisting of oxygen, sulfur, selenium and nitrogen, the remaining ring atoms being carbon;

A is a group promoting the adsorption of the nucleating agent to the silver halide grains;

m and n are 1 or 2; and

$R^1$, $R^2$ and $R^3$ are independently selected from the group consisting of hydrogen alkyl, aryl, alkaryl and aralkyl, and $R^1$ and $R^3$ are additionally independently selected from the group consisting of acyl, alkenyl and alkynyl, the aliphatic portions of such groups containing up to 5 carbon atoms and the aromatic portions of such groups containing 6 to 10 carbon atoms.

2. A silver halide emulsion according to claim 1, characterized in that said silver halide grains are predominantly silver bromide and contain metal dopants occluded therein, which grains when coated on a photographic support, exposed to a light intensity scale, and developed for 5 minutes at 25°C in test surface developer provide (a) a density of less than 0.4 and (b) a density of at least 0.5 less than when this testing procedure is repeated modifying the test surface developer by the inclusion of 0.5 gram per liter of potassium iodide, the test surface developer being of the following composition:

| | |
|---|---|
| Water | 500.0 ml |
| N-methyl-*p*-aminophenol sulfate | 2.5 g |
| Sodium sulfite, desiccated | 30.0 g |
| Hydroquinone | 2.5 g |
| Sodium metaborate | 10.0 g |
| Potassium bromide | 0.5 g |
| Water to make | 1.0 liter |

3. A silver halide emulsion according to claims 1 or 2, characterized in that said nucleating agent is present in a concentration of from $10^{-5}$ to $10^{-2}$ mole per mole of silver halide.

4. A silver halide emulsion according to any of claims 1, 2 or 3, characterized in that said nucleating agent is of the formula

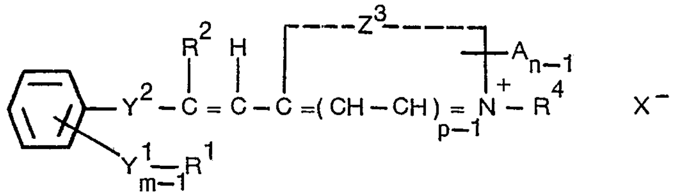

wherein

m and p are 1 or 2;

$Y^1$ and $Y^2$ are independently selected from the group consisting of divalent oxygen atom, a divalent sulfur atom and

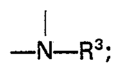

$Z^3$ completes a heterocycle forming one 5- or 6-membered ring containing at least one non-metallic heteroatom in the ring selected from the group consisting of oxygen, sulfur, selenium and nitrogen, the remaining ring atoms being carbon;

$R^1$, $R^2$ and $R^3$ are independently selected from the group consisting of hydrogen, alkyl, aryl, alkaryl and aralkyl, $R^1$ and $R^3$ are additionally independently selected from the group consisting of acyl, alkenyl and alkynyl, and $R^4$ is independently selected from the group consisting of alkyl, aryl, alkaryl, alkenyl and aralkyl the aliphatic portions of such groups containing up to 5 carbon atoms and aromatic portions of such groups containing 6 to 10 carbon atoms; and

$X^-$ is an acid anion, if required for charge neutrality; and

A is a group promoting the adsorption of the nucleating agent to the silver halide grains.

5. A silver halide emulsion according to claim 4, characterized in that $Y^2$ is oxygen.

6. A silver halide emulsion according to any of claims 1 to 5, characterized in that said nucleating agent is an alkylaminophenoxyvinyl-3-alkylbenzothiazolium salt.

7. A silver halide emulsion according to claim 4, characterized in that $Y^2$ is

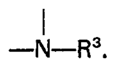

8. A silver halide emulsion according to any of claims 1 to 4, and 7 characterized in that said nucleating agent is selected from the group consisting of an N-alkyl substituted benzothiazolium, quinolinium, benzoxazolium, benzoselenazolium and benzimidazolium salt which is β-anilinovinyl substituted.

9. A silver halide emulsion according to any of claims 1, 2 or 3, characterized in that said nucleating agent is a thioureido substituted benzothiazolium salt which is N-substituted with an alkyl group and substituted in its 2-position with β-anilinovinyl group.

10. A silver halide emulsion according to claims 1 or 2, characterized in that the nucleating agent is selected from the group consisting of 2-[β-anilinovinyl]-3-methylbenzothiazolium *p*-toluenesulfonate, 2-[β-(N-methylanilino)vinyl]-3-methylbenzothiazolium *p*-toluenesulfonate, 2-[β-(*o*-methylthio-N-methylanilino)vinyl]-1-methylquinolinium *p*-toluenesulfonate, 2-[β-(N-propargylanilino)vinyl]-3-ethylbenzoxazolium bromide, 2-[β-(*p*-N-methylaminophenoxy)vinyl]-3-methylbenzothiazolium chloride, 2-[β-(*o*-methoxy-N-methylanilino)vinyl]-3-methylbenzothiazolium *p*-toluenesulfonate, 2-[β-(N-propargylanilino)vinyl]-3-ethylbenzoselenazolium bromide, 1-ethyl-2-[β-N-propargylanilinovinyl]-1,3-diethyl-5,6-dichlorobenzimidazolium bromide, 2-[β-(*o*-propargylthio-N-propargylthio-N-propargylanilino)vinyl]-3-methyl-benzothiazolium bromide, 2-[β-(*o*-methylthio-N-propargylanilino)vinyl]-3-methylbenzothiazolium p-toluenesulfonate, 2-[β-methyl-β-(*o*-propargylthio-N-methylanilino)vinyl]-3-methylbenzothiazolium bromide, 2-[β-N-propargylanilinovinyl]-3-methylbenzothiazolium bromide, 2-[β-(N-methylanilino)vinyl]-3-methyl-6-acetamidobenzothiazolium *p*-toluenesulfonate and 2-[β-(N-methylanilino)vinyl]-3-methyl-6-(N'-ethylthioureido)benzothiazolium bromide.

11. A photographic element comprised of a support having coated thereon at least one silver halide emulsion, containing silver halide grains capable of forming an internal latent image and a nucleating agent characterized in that said nucleating agent has the formula

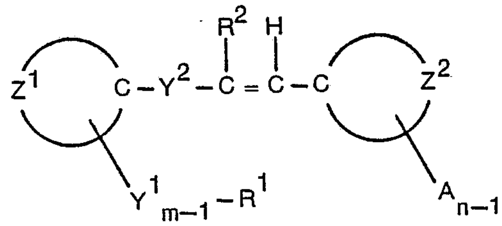

wherein

$Z^1$ represents the atoms completing an aromatic carbocyclic nucleus of from 6 to 10 nuclear carbon atoms;

$Y^1$ and $Y^2$ are independently selected from the group consisting of a divalent oxygen atom, a divalent sulfur atom and

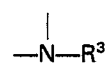

$Z^2$ represents the atoms completing a heterocyclic nucleus comprising a 5- or 6-membered ring containing at least one non-metallic heteroatom in the ring selected from the group consisting of oxygen, sulfur, selenium and nitrogen, the remaining ring atoms being carbon;

A is a group promoting the adsorption of the nucleating agent to the silver halide grains;

m and n are 1 or 2; and

$R^1$, $R^2$ and $R^3$ are independently selected from the group consisting of hydrogen, alkyl, aryl, alkaryl and aralkyl and $R^1$ and $R^3$ are additionally independently selected from the group consisting of acyl, alkenyl and alkynyl, the aliphatic portions of such groups containing up to 5 carbon atoms and the aromatic portions of such groups containing 6 to 10 carbon atoms.

**Revendications**

1. Emulsion aux halogénures d'argent comprenant des grains d'halogénures d'argent aptes à former une image latente interne et un agent de nucléation, caractérisée en ce que cet agent de nucléation est un composé de formule:

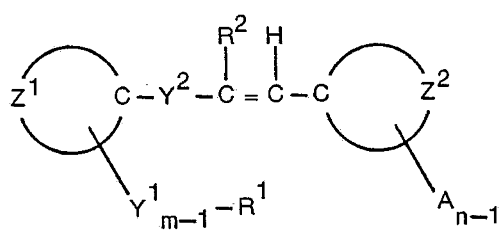

où:

$Z^1$ représente les atomes pour compléter un carbocycle aromatique de 6 à 10 atomes de carbone dans le cycle,

$Y^1$ et $Y^2$ sont, indépendamment l'un de l'autre, choisis dans le groupe constitué d'un atome d'oxygène bivalent, un atome de soufre bivalent et un groupe bivalent $—NR^3—$,

$Z^2$ représente les atomes complétant un hétérocycle comprenant un cycle à 5 ou 6 chaînons qui contient dans le cycle, au moins un hétéroatome non métallique choisi dans le groupe constitué par l'oxygène, le soufre, le sélénium et l'azote, les autres atomes du cycle étant des atomes de carbone,

A est un groupe favorisant l'adsorption de l'agent de nucléation sur les grains d'halogénures d'argent,

m et n sont 1 ou 2, et,

$R^1$, $R^2$ et $R^3$ sont, indépendamment l'un de l'autre, choisis dans le groupe constitué par l'hydrogène, un radical alkyle, aryle, alkaryle et aralkyle et $R^1$ et $R^3$ sont en outre, indépendamment l'un de l'autre, choisis dans le groupe constitué par un radical acyle, alkényle et alkynyle, les parties aliphatiques de ces radicaux

contenant jusqu'à 5 atomes de carbone et les parties aromatiques contenant 6 à 10 atomes de carbone.

2. Emulsion aux halogénures d'argent conforme à la revendication 1, caractérisée en ce que ces grains d'halogénures d'argent sont principalement du bromure d'argent à l'intérieur desquels sont occlus des dopants métalliques, ces grains appliqués sur un support photographique, exposés à une échelle de teinte et développés pendant 5 minutes, à 25°C, dans un révélateur superficiel produisant (a) une densité inférieure à 0,4 et (b) une densité inférieure d'au moins 0,5 à celle qu'on obtient lorsqu'on reproduit cette procédure d'essai en modifiant le révélateur superficiel par l'incorporation de 0,5 g/l d'iodure de potassium, le révélateur superficiel ayant la composition suivante:

| | |
|---|---|
| Eau | 500,0 ml |
| Sulfate de N-méthyl-p-aminophénol | 2,5 g |
| Sulfite de sodium anhydre | 30,0 g |
| Hydroquinone | 2,5 g |
| Métaborate de sodium | 10,0 g |
| Bromure de potassium | 0,5 g |
| Eau q.s.p. | 1,0 l |

3. Emulsion aux halogénures d'argent conforme à la revendication 1 ou 2, caractérisée en ce qu'elle contient $10^{-5}$ à $10^{-2}$ mole d'argent de nucléation par mole d'halogénures d'argent.

4. Emulsion aux halogénures d'argent conforme à l'une quelconque des revendications 1 à 3, caractérisée en ce que l'agent de nucléation correspond à la formule suivante:

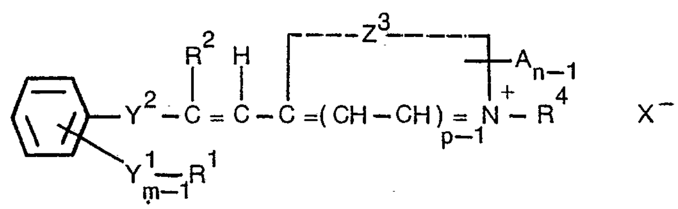

où:

m, n et p sont 1 ou 2,

$Y^1$ et $Y^2$ représentent indépendamment un atome d'oxygène bivalent, de soufre bivalent ou un radical bivalent $—NR^3—$,

$Z^3$ complète un hétérocycle comprenant un cycle à 5 ou 6 chaînons avec, dans ce cycle, au moins un hétéroatome non métallique choisi dans le groupe constitué par l'oxygène, le soufre, le sélénium et l'azote, les autres atomes du cycle étant des atomes de carbone,

$R^1$, $R^2$ et $R^3$ sont indépendamment choisi dans le groupe constitué par l'hydrogène, un radical alkyle, aryle, alkaryle et aralkyle, $R^1$ et $R^3$ sont, en outre, indépendamment choisi dans le groupe constitué par un radical acyle, alkényle et alkynyle et $R^4$ est indépendamment choisi dans le groupe constitué par un radical alkyle, aryle alkaryle, alkényle et aralkyle, les parties aliphatiques de ces radicaux contenant jusqu'à 5 atomes de carbone et les parties aromatiques contenant 6 à 10 atomes de carbone,

$X^-$ est un anion, si cela est nécessaire pour la neutralité des charges, et,

A est un groupe favorisant l'adsorption de l'agent de nucléation sur les grains d'halogénures d'argent.

5. Emulsion aux halogénures d'argent conforme à la revendication 4, caractérisée en ce que $Y^2$ est l'oxygène.

6. Emulsion aux halogénures d'argent conforme a l'une quelconque des revendications 1 à 5, caractérisée en ce que l'agent de nucléation est un sel d'alkylaminophénoxy-vinyl-3-alkylbenzothiazolium.

7. Emulsion aux halogénures d'argent conforme à la revendication 4, caractérisée en ce que $Y^2$ est $—NR^3—$.

8. Emulsion aux halogénures d'argent conforme à l'une quelconque des revendications 1 à 4 et 7, caractérisée en ce que l'agent de nucléation est choisi dans le groupe constitué par les sels de benzothazolium, quinoléinium, benzoxazolium, benzosélénazolium et benzimidazolium portant un substituant N-alkyle et un substituant β-anilinovinyle.

9. Emulsion aux halogénures d'argent conforme à l'une quelconque des revendications 1 à 3, caractérisée en ce que l'agent de nucléation est un sel de benzothiazolium, substitué par un groupe thiouréido, et qui porte un substituant N-alkyle et un substituant β-anilinovinyle en position 2.

10. Emulsion aux halogénures d'argent conforme à l'une des revendications 1 ou 2, caractérisée en ce que l'agent de nucléation est choisi dans le groupe constitué par le p-toluènesulfonate de 2-[β-anilinovinyl]-3-méthylbenzothiazolium, le p-toluènesulfonate de 2-[β-(N-méthylanilino)vinyl]-3-méthylbenzothiazolium, le p-toluènesulfonate de 2-[β-(*o*-méthylthio-N-méthylanilino)vinyl]-1-méthylquinolinium, le bromure de 2-[β-(N-propargylanilino)vinyl]-3-éthylbenzoxazolium, le chlorure de 2-[β-(*p*-N-méthylaminophénoxy)vinyl]-3-méthylbenzothiazolium, le *p*-toluènesulfonate de 2-[β-(*o*-méthoxy-N-méthylanilino)vinyl]-3-méthylbenzothiazolium, le bromure de 2-[β-(N-propargylanilino)vinyl]-3-éthylbenzosélénazolium, le bromure de 1-éthyl-2-[β-N-propargylanilinovinyl]-1,3-diéthyl-5,6-dichlorobenzimidazolium, le bromure de 2-[β-(*o*-propargylthio-N-propargylanilino)vinyl]-3-méthyl-benzothiazolium, le *p*-toluènesulfonate de 2-[β-(*o*-méthylthio-N-propargylanilino)vinyl]-3-méthylbenzothiazolium, le bromure de 2-[β-méthyl-β-(*o*-propargylthio-N-méthylanilino)-vinyl]-3-méthylbenzothiazolium, le bromure de 2-[β-N-propargylanilinovinyl]-3-méthylbenzothiazolium, le *p*-toluènesulfonate de 2-[β-méthylanilino)vinyl]-3-méthyl-6-acétamidobenzothiazolium et le bromure de 2-[β-(N-méthylanilino)vinyl]-3-méthyl-6-(N'-éthylthioureido)benzothiazolium.

11. Produit photographique comprenant un support sur lequel est appliquée au moins une couche d'émulsion aux halogénures d'argent qui contient des grains d'halogénures d'argent aptes à former une image latente interne et un agent de nucléation, ce produit photographique étant caractérisé en ce que cet agent de nucléation correspond à la formule:

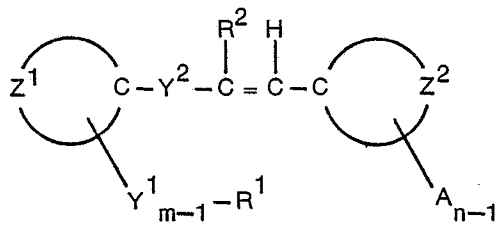

où:

$Z^1$ représente les atomes pour compléter un carbocycle aromatique de 6 à 10 atomes de carbone dans le cycle,

$Y^1$ et $Y^2$ sont, indépendamment l'un de l'autre, choisis dans le groupe constitué d'un atome d'oxygène bivalent, un atome de soufre bivalent et un groupe bivalent $—NR^3—$,

$Z^2$ représente les atomes complétant un hétérocycle comprenant un cycle à 5 ou 6 chaînons qui contient dans le cycle, au moins un hétéroatome non métallique choisi dans le groupe constitué par l'oxygène, le soufre, le sélénium et l'azote, les autres atomes du cycle étant des atomes de carbone,

A est un groupe favorisant l'adsorption de l'agent de nucléation sur les grains d'halogénures d'argent,

m et n sont 1 ou 2, et,

$R^1$, $R^2$ et $R^3$ sont, indépendamment l'un de l'autre, choisis dans le groupe constitué par l'hydrogène, un radical alkyle, aryle, alkaryle et aralkyle et $R^1$ et $R^3$ sont en outre, indépendamment l'un de l'autre, choisis dans le groupe constitué par un radical acyle, alkényle et alkynyle, les parties aliphatiques de ces radicaux contenant jusqu'à 5 atomes de carbone et les parties aromatiques contenant 6 à 10 atomes de carbone.

**Patentansprüche**

1. Silberhalogenidemulsion mit Silberhalogenidkörnern, die ein latentes Innenbild zu erzeugen vermögen und einem Keimbildner, dadurch gekennzeichnet, daß der Keimbildner eine Verbindung der Formel:

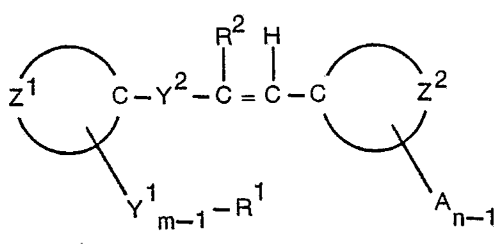

ist, in der bedeuten:

$Z^1$ die Atome, die einen aromatischen carbocyclischen Kern mit 6 bis 10 Kernkohlenstoffatomen vervollständigen;

$Y^1$ und $Y^2$ unabhängig voneinander ein zweiwertiges Sauerstoffatom, ein zweiwertiges Schwefelatom oder einen Rest der Formel

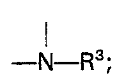

$Z^2$ die Atome, die einen heterocyclischen Kern mit einem 5- oder 6-gliedrigen Ring mit mindestens einem nichtmetallischen Heteroatom im Ring bestehend aus einem Sauerstoff-, Schwefel-, Selen- oder Stickstoffatom und zum Rest Kohlenstoffatomen, vervollständigen;

A eine Gruppe, die die Adsorption des Keimbildners an die Silberhalogenidkörner fördert;

m und n = 1 oder 2 und

$R^1$, $R^2$ und $R^3$ unabhängig voneinander Wasserstoffatome, Alkyl-, Aryl-, Alkaryl- oder Aralkylgruppen und $R^1$ und $R^3$ zusätzlich unabhängig voneinander Acyl-, Alkenyl- oder Alkynylgruppen, wobei die aliphatischen Teile dieser Gruppen bis zu 5 C-Atome und die aromatischen Teile dieser Gruppen 6 bis 10 C-Atome aufweisen.

2. Silberhalogenidemulsion nach Anspruch 1, dadurch gekennzeichnet, daß die Silberhalogenidkörner zum überwiegenden Teil aus Silberbromid bestehen, eingeschlossene Metalldotiermittel enthalten und nach Auftragen auf einen photographischen Träger, einer Lichtskalenexponierung und 5 Minuten langer Entwicklung bei 25°C in einem Test-Oberflächenentwickler (a) eine Dichte von weniger als 0,4 erzeugen und (b) eine Dichte, die um mindestens 0,5 geringer ist als bei Wiederholung des Testverfahrens mit einem Test-Oberflächenentwickler, der durch Zusatz von 0,5 g Kaliumjodid pro Liter modifiziert worden ist, wobei der Test-Oberflächenentwickler die folgende Zusammensetzung hat:

| | |
|---|---|
| Wasser | 500,0 ml |
| N-Methyl-*p*-aminophenolsulfat | 2,5 g |
| Natriumsulfit, entwässert | 30,0 g |
| Hydrochinon | 2,5 g |
| Natriummetaborat | 10,0 g |
| Kaliumbromid | 0,5 g |
| Mit Wasser aufgefüllt auf | 1,0 l |

3. Silberhalogenidemulsion nach Ansprüchen 1 oder 2, dadurch gekennzeichnet, daß der Keimbildner in einer Konzentration von $10^{-5}$ bis $10^{-2}$ Molen pro Mol Silberhalogenid vorliegt.

4. Silberhalogenidemulsion nach einem der Ansprüche 1, 2 oder 3, dadurch gekennzeichnet, daß der Keimbildner der folgenden Formel entspricht:

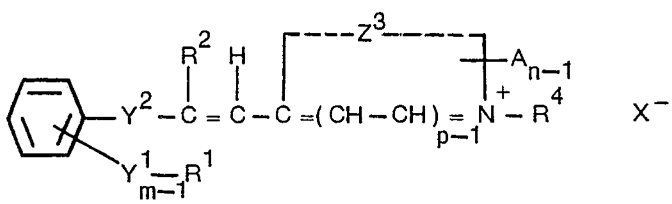

in der bedeuten:

m und p = 1 oder 2;

$Y^1$ und $Y^2$ unabhängig voneinander jeweils ein divalentes Sauerstoffatom, ein divalentes Schwefelatom oder ein Rest der Formel

$$-N-R^3;$$

$Z^3$ die zur Vervollständigung eines heterocyclischen Kernes mit einem 5- oder 6-gliedrigen Ring mit mindestens einem nicht-metallischen Heteroatom im Ring erforderlichen Atome, wobei das Heteroatom aus einem Sauerstoff-, Schwefel-, Selen- oder Stickstoffatom besteht und die restlichen Atome Kohlenstoffatome sind;

$R^1$, $R^2$ und $R^3$ unabhängig voneinander, Wasserstoffatome oder Alkyl-, Aryl-, Alkaryl- oder Aralkylgruppen und $R^1$ und $R^3$ zusätzlich Acyl-, Alkenyl- und Alkynylgruppen und $R^4$ davon unabhängig eine Alkyl-, Aryl-, Alkaryl-, Alkenyl- oder Aralkylgruppe, wobei die aliphatischen Reste dieser Gruppen bis zu 5 C-Atome und die aromatischen Reste dieser Gruppen 6 bis 10 C-Atome aufweisen;

$X^-$ ein Säureanion, wenn für die Ladungsneutralisation erforderlich, und

A eine Gruppe, die die Adsorption des Keimbildners an die Silberhalogenidkörner fördert.

5. Silberhalogenidemulsion nach Anspruch 4, dadurch gekennzeichnet, daß $Y^2$ für ein Sauerstoffatom steht.

6. Silberhalogenidemulsion nach einem der Ansprüche 1 bis 5, dadurch gekennzeichnet, daß der Keimbildner ein Alkylaminophenoxyvinyl-3-alkylbenzothiazoliumsalz ist.

7. Silberhalogenidemulsion nach Anspruch 4, dadurch gekennzeichnet, daß $Y^2$ für eine Gruppe der Formel

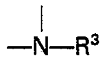

steht.

8. Silberhalogenidemulsion nach einem der Ansprüche 1 bis 4 und 7, dadurch gekennzeichnet, daß der Keimbildner ausgewählt ist aus einem N-Alkyl-silbstituierten Benzothiazolium, Chinolinium-, Benzoxazolium-, Benzoselenazolium oder Benzimidazoliumsalz, das ß-Anilinovinyl-substituiert ist.

9. Silberhalogenidemulsion nach einem der Ansprüche 1, 2 oder 3, dadurch gekennzeichnet, daß der Keimbildner ein Thioureido-substituiertes Benzothiazoliumsalz ist, das durch eine Alkylgruppe N-substituiert und in seiner 2-Position durch eine β-Anilinovinylgruppe substituiert ist.

10. Silberhalogenidemulsion nach Ansprüchen 1 oder 2, dadurch gekennzeichnet, daß der Keimbildner ausgewählt ist aus: 2-[β-Anilinovinyl]-3-methylbenzothiazolium-*p*-toluolsulfonat, 2-[β-(N-Methylanilino)vinyl]-3-methylbenzothiazolium-*p*-toluolsulfonat, 2-[β-(*o*-Methylthio-N-methylanilino)vinyl]-1-methylchinolinium-p-toluolsulfonat, 2-[β-N-Propargylanilino)vinyl]-3-ethylbenzoxazoliumbromid, 2-[β-(*p*-N-Methylaminophenoxy)vinyl]-3-methylbenzothiazoliumchlorid, 2-[β-(*o*-Methoxy-N-methylanilino)vinyl]-3-methylbenzothiazolium-*p*-toluolsulfonat, 2-[β-(N-Propargylanilino)vinyl]-3-ethylbenzoselenazoliumbromid, 1-Ethyl-2-[β-N-propargylanilinovinyl]-1,3-diethyl-5,6-dichlorobenzimidazoliumbromid, 2-[β-(*o*-Propargylthio-N-propargylanilino)vinyl]-3-methylbenzothiazoliumbromid, 2-[β-(*o*-Methylthio-N-propargylanilino)vinyl]-3-methylbenzothiazolium-p-toluolsulfonat, 2-[β-Methyl-β-(o-propargylthio-N-methylanilino)-vinyl]-3-methylbenzothiazoliumbromid, 2-[β-N-Propargylanilinovinyl]-3-methylbenzothiazolium bromid, 2-[β-(N-Methylanilino)vinyl]-3-methyl-6-acetamidobenzothiazolium-*p*-toluolsulfonat und 2-[β-(N-Methylanilino)vinyl]-3-methyl-6-(N'-ethylthioureido)benzothiazoliumbromid.

11. Photographisches Element aus einem Träger, auf den mindestens eine Silberhalogenidemulsion mit Silberhalogenidkörnern, die ein latente Innenbild zu liefern vermögen und ein Keimbildner aufgetragen sind, dadurch gekennzeichnet, daß der Keimbildner der folgenden Formel entspricht:

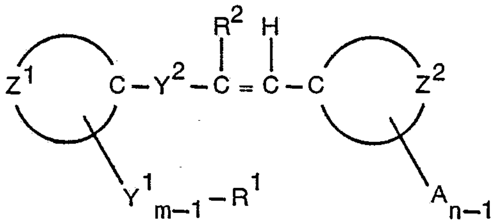

in der bedeuten:

$Z^1$ die Atome, die einen aromatischen carbocyclischen Kern mit 6 bis 10 Kernkohlenstoffatomen vervollständigen;

$Y^1$ und $Y^2$ unabhängig voneinander ein zweiwertiges Sauerstoffatom, ein zweiwertiges Schwefelatom oder einen Rest der Formel

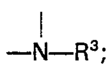;

$Z^2$ die Atome, die einen heterocyclischen Kern mit einem 5- oder 6-gliedrigen Ring mit mindestens einem nichtmetallische Heteroatom im Ring bestehend aus einem Sauerstoff-, Schwefel-, Selen- oder Stickstoffatom und zum Rest Kohlenstoffatomen, vervollständigen;

A eine Gruppe, die die Adsorption des Keimbildners an die Silberhalogenidkörner fördert;

m und n = 1 oder 2 und

$R^1$, $R^2$ und $R^3$ unabhängig voneinander Wasserstoffatome, Alkyl-, Aryl-, Alkaryl- oder Aralkylgruppen und $R^1$ und $R^3$ zusätzlich unabhängig voneinander Acyl-, Alkenyl- oder Alkynylgruppen, wobei die aliphatischen Teile dieser Gruppen bis zu 5 C-Atome und die aromatischen Teile dieser Gruppe 6 bis 10 C-Atome aufweisen.